# EUROPÄISCHE PATENTSCHRIFT

(11) **EP 0 894 083 B1**
(45) Veröffentlichungstag und Bekanntmachung des Hinweises auf die Patenterteilung: **03.11.1999**
(21) Anmeldenummer: 97954423.6
(22) Anmeldetag: 18.12.1997
(51) Int. Cl.: C07C 279/14, A61K 31/205, A23L 1/305

(54) **KREATIN-PYRUVATE UND VERFAHREN ZU DEREN HERSTELLUNG**
CREATINE PYRUVATES AND METHOD FOR THEIR PRODUCTION
CREATINE PYRUVATES ET LEUR PROCEDE DE PREPARATION

(30) Priorität: 20.12.1996 DE 19653225; 11.07.1997 US 893423
(43) Veröffentlichungstag der Anmeldung: 03.02.1999
(73) Patentinhaber: SKW Trostberg Aktiengesellschaft, 83308 Trostberg (DE)
(72) Erfinder: PISCHEL, Ivo, D-83342 Tacherting (DE); WEISS, Stefan, D-83308 Trostberg (DE)
(74) Vertreter: Weiss, Wolfgang, Dipl.-Chem. Dr.
(86) Internationale Anmeldenummer: EP9707121
(87) Internationale Veröffentlichungsnummer: WO9828263

(56) Entgegenhaltungen:
- EP-A- 0 108 820
- WO-A-96/04240
- FR-M- 4 508

## Beschreibung

Die vorliegende Anmeldung betrifft Kreatin-pyruvate und deren Herstellung, wobei es sich um wasserfreie oder wasserhaltige Salze der Brenztraubensäure mit Kreatin und Mischungen dieser Salze mit Kreatin oder Brenztraubensäure handelt.

Es ist bekannt, daß Salze der Brenztraubensäure, die als Pyruvate bezeichnet werden, wertvolle physiologische und therapeutische Eigenschaften für die Behandlung von verschiedenen Krankheiten, wie z.B. Fettsucht und Übergewicht, aufweisen und auch zur Verhinderung der Bildung von freien Radikalen sowie zur Ausdauersteigerung verwendet werden können (vgl. hierzu US 5,508,308, US 5,480,909, US 5,472,980, US 5,395,822, US 5, 312,985, US 5,283,260, US 5,256,697, US 4,548,937 sowie US 4,351,835).

Entsprechend dem Stand der Technik sind Alkali- und Erdalkali-pyruvate bekannt, wobei Natrium- und Kalium-pyruvat aufgrund ihres Gehaltes an Natrium- bzw. Kaliumionen für therapeutische Anwendungen und als Nahrungsmittelergänzungszusätze jedoch nicht geeignet sind. Magnesium- und Calcium-pyruvat sind zwar physiologisch unbedenklich, allerdings weisen diese Salze den entscheidenden Nachteil auf, daß sie nicht ausreichend lagerstabil sind, da Magnesium- und Calciumionen die Zersetzung von Brenztraubensäure und Pyruvationen stark beschleunigen, wobei u.a. Dimere, Polymere und cyclische Verbindungen gebildet werden.

In EP-A-0-108 820 sind Infusionslösungen zur Herztherapie offenbart, die dadurch gekennzeichnet sind, daß sie Brenztraubensäure und/oder deren physiologisch verträgliche Salze, z.B. das Natrium-, Kalium-, Calcium-, Magnesium- oder Ammoniumsalz enthalten. In FR-M-4508 wird das Pyridoxin-Salz der Brenztraubensäure zur Herstellung pharmazeutisch wirksamer Zusammensetzungen, die zur Therapie verschiedener Krankheitszustände, wie z.B. Appetitlosigkeit, einsetzbar sind, offenbart.

Der vorliegenden Erfindung lag daher die Aufgabe zugrunde, Formen der Brenztraubensäure zu entwickeln, die physiologisch unbedenklich sind und gleichzeitig eine ausreichende Lagerstabilität besitzen.

Diese Aufgabe wurde erfindungsgemäß durch die Bereitstellung von Kreatin-pyruvaten der Formel (I)

(Kreatin)ₓ(Pyruvat)_{y}(H₂O)ₙ (I)

gelöst,
wobei
x = 1 bis 100
y = 1 bis 10 und
n = 0 bis 10 bedeuten.

In den Verbindungen der Formel (I) kann - entsprechend den stöchiometrischen Erfordernissen - Kreatin in ungeladener Form oder als Kation und Pyruvat als Brenztraubensäure oder als Anion vorliegen.

Es hat sich nämlich überraschenderweise gezeigt, daß die erfindungsgemäßen Kreatin-pyruvate eine gute Lagerstabilität aufweisen, obwohl Brenztraubensäure eine sehr instabile 2-Oxocarbonsäure ist und sich die bekannten Salze des Kreatins leicht unter Bildung von Kreatinin zersetzen. Da Kreatin als inneres Salz vorliegt und nur eine schwache Base darstellt, konnte nicht damit gerechnet werden, daß man stabile Kreatinsalze von Monocarbonsäuren herstellen kann. Entsprechend dem Stand der Technik sind nämlich bislang nur Kreatinsalze von starken Di- und Polycarbonsäuren bekannt (vgl. WO 96/04 240).

Die erfindungsgemäßen Kreatin-pyruvate der allgemeinen Formel (I) enthalten das physiologisch unbedenkliche Kreatin-Kation der Formel (II)

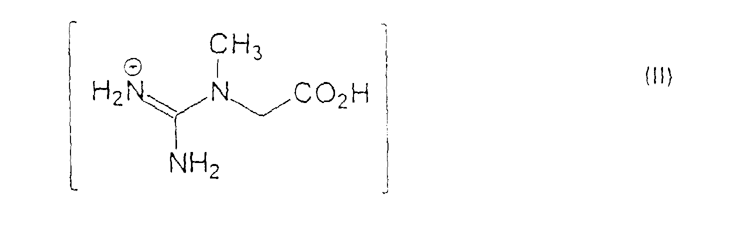

Kreatin ist nicht nur eine körpereigene Substanz und ein wertvolles Nahrungsergänzungsmittel, sondern besitzt auch wertvolle therapeutische Eigenschaften. Kreatin ist seit über 100 Jahren als Muskelsubstanz bekannt, wobei es dem Muskel als Energiequelle dient. In einer Reihe von wissenschaftlichen Arbeiten wurde gezeigt, daß die Einnahme von Kreatin zu einer Steigerung der Muskelmasse und Muskelleistung führen kann. Es gibt auch wissenschaftliche Erkenntnisse, daß die Bauchspeicheldrüse unter dem Einfluß von Kreatin vermehrt Insulin freisetzt. Insulin fördert die Aufnahme von Glukose und Aminosäuren in die Muskelzelle und regt die Proteinsynthese an. Außerdem vermindert Insulin auch die Proteinabbaurate.

Das Pyruvat-Anion in den erfindungsgemäßen Kreatin-pyruvaten liegt normalerweise in einer Struktur gemäß Formel (III) vor.

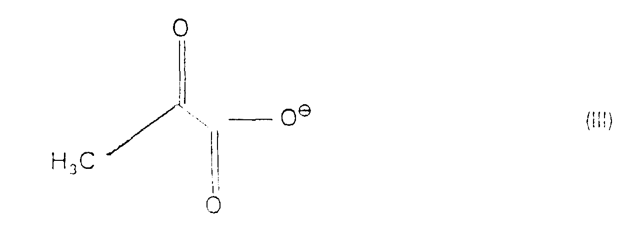

In den Kristallwasser-haltigen Kreatin-pyruvaten kann das Pyruvat-Anion auch in der 2,2-Dihydroxy-Form entsprechend der Formel (IV) vorliegen:

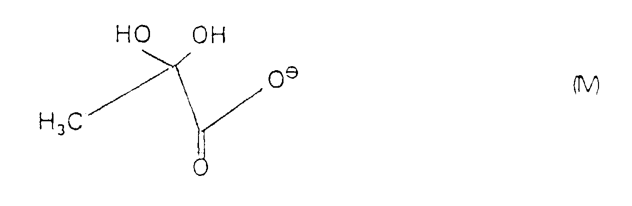

Die erfindungsgemäßen Kreatin-pyruvate umfassen Salze, welche das Kreatin-Kation und das Pyruvat-Anion bzw. das 2,2-Dihydroxypropionat-Anion vorzugsweise im Molverhältnis 1 : 1 oder annähernd im Molverhältnis 1 : 1 enthalten. Gegebenenfalls können die erfindungsgemäßen Verbindungen auch Mischungen dieser Salze mit Kreatin oder Brenztraubensäure darstellen.

Die Herstellung der erfindungsgemäßen Kreatin-pyruvate kann durch relativ einfache Umsetzung von Kreatin mit Brenztraubensäure im Temperaturbereich von -10 bis 90 °C, vorzugsweise im Temperaturbereich von 10 bis 30 °C, durchgeführt werden. Hierbei werden Kreatin und Brenztraubensäure im Molverhältnis von 100 : 1 bis 1 : 10 und vorzugsweise 5 : 1 bis 1 : 2 umgesetzt. Kreatin kann hierbei in wasserfreier Form, als Monohydrat oder als feuchtes Produkt verwendet werden. Die Brenztraubensäure kann als wasserfreie Säure oder als wäßrige Lösung zum Einsatz gelangen.

Die Umsetzung kann in Abwesenheit oder in Gegenwart eines Löse- oder Verdünnungsmittels durchgeführt werden, wobei als Löse- oder Verdünnungsmittel eine breite Palette von polaren Lösemitteln geeignet sind. Bevorzugt werden Alkohole (wie Methanol, Ethanol, Isopropanol, Cyclohexanol), Ether (wie Diethylether, Tetrahydrofuran, 1,4-Dioxan, Ethylendimethylether), Ketone (wie Aceton, Methylethylketon, Cyclohexanon) oder Ester (wie Essigsäuremethylester, Essigsäureethylester, Ameisensäureethylester) oder Gemische davon verwendet. Die Umsetzung kann hierbei in den bekannten verfahrenstechnischen Apparaten wie in Mischern, Schaufeltrocknern und Rührbehältern erfolgen.

Die Kristallwasser-haltigen Kreatin-pyruvate sind durch Zusatz von Wasser während oder nach der Umsetzung von Brenztraubensäure mit Kreatin oder/und durch Verwendung von wäßrigem Kreatin oder/und wäßriger Brenztraubensäure erhältlich. Im Rahmen der vorliegenden Erfindung ist es auch möglich, bei oder nach der Herstellung noch andere Stoffe wie pharmazeutische Formulierungshilfsmittel, Vitamine, Mineralstoffe, Spurenelemente, Kohlenhydrate wie Glukose, Dextrose, Maltose oder Aminosäuren wie L-Carnitin oder andere Nahrungsergänzungsmittel zuzusetzen.

Ein Gegenstand der Erfindung sind somit auch physiologisch verträgliche Zusammensetzungen, die Kreatin-pyruvate zusammen mit mindestens einer weiteren physiologisch verträglichen Substanz, ausgewählt aus der Gruppe umfassend pharmazeutische Hilfs- oder Trägerstoffe, Vitamine, Mineralstoffe, Kohlenhydrate, Aminosäuren oderandere Nahrungsergänzungsmittel enthalten.

Aufgrund ihrer optimalen Eigenschaften wie physiologische Unbedenklichkeit, hohe Lagerstabilität sowie gute Wasserlöslichkeit und hohe Bioverfügbarkeit eignen sich die erfindungsgemäßen Kreatin-pyruvate hervorragend für therapeutische Anwendungen in der Medizin und als Nahrungsergänzungsmittelzusätze, wobei diese die wertvollen biologischen und medizinischen Eigenschaften sowohl von Pyruvaten als auch von Kreatin besitzen.

Überraschenderweise zeigen die erfindungsgemäßen Kreatin-pyruvate bei Anwendung in der Medizin und als Nahrungsergänzungsmittelzusätze deutliche synergistische Effekte. Sie eignen sich hierbei in ganz besonderer Weise zur Behandlung von Sauerstoffmangelzuständen (lschämie) sowie Übergewicht und Fettsucht, da der Abbau von Muskelmasse während der Behandlung vermindert wird, wobei der muskelaufbauende Effekt von Kreatin-pyruvat besonders bei Diätkuren große Vorteile mit sich bringt. Sie verhindern ebenfalls die Bildung freier Radikale und sind in der Lage, freie Radikale oder oxidierende Sauerstoffspezies abzufangen. Die synergistischen Effekte zeigen sich auch insbesondere bei der Verwendung von Kreatin-pyruvat im Sportbereich zur Steigerung der Ausdauerleistung.

Die nachfolgenden Beispiele sollen die Erfindung veranschaulichen.

### Beispiele

### Beispiel 1

26,4 g (0,3 mol) Brenztraubensäure werden bei Raumtemperatur in 100 ml Ethylacetat gelöst. Zu dieser Lösung werden 26,2 g (0,2 mol) Kreatin gegeben und das Gemisch 4 Stunden gerührt. Anschließend wird die weiße, feinkristalline Masse abfiltriert und der Rückstand zweimal mit 25 ml Ethylacetat gewaschen. Das Produkt wird 4 Stunden bei 50°C im Vakuumtrockenschrank getrocknet. Die Ausbeute beträgt 95,0%. Das Kreatin-pyruvat (1:1) schmilzt bei 106 bis 110°C unter Zersetzung (Kapillare).

Elementaranalyse C₇H₁₃N₃O₅: ber.: C 38,36 %, H 5,94%, N 19,18%; gef.: C 38,23%, H 6,06%, N 19,28%; IR (KBr) [1/cm]: 620, 829, 880, 976, 1049, 1110, 1177, 1209, 1269, 1354, 1404, 1605, 1663, 1697, 1734, 1763, 2518, 2593, 3147, 3397; ¹H-NMR (D₂O, 300 MHz): δ = 2,34 (s, 3H, MeCO), 3,08 (s, 3H, Me-N), 4,06 (s, 2H, CH₂); HPLC-Gehalt: Kreatin 59,8%, Brenztraubensäure 40,2%.

### Beispiel 2

26,2 g (0,2 mol) Kreatin werden mit 17,6 (0,2 mol) Brenztraubensäure in einer Reibschale vermischt. Das Gemisch wird zunehmend zäher und erstarrt schließlich zu einer weißen, feinkristallinen Masse. Die Ausbeute ist quantitativ (> 99%). Der Schmelzpunkt des Kreatin-pyruvats (1:1) beträgt 109 bis 114°C unter Zersetzung (Kapillare).

### Beispiel 3

29,8 g (0,2 mol) Kreatin-Monohydrat werden mit 35,2 g (0,4 mol) Brenztraubensäure in einem Becherglas innig vermischt. Das Gemisch wird sich selbst überlassen, wobei es schließlich zu einer weißen, feinkristallinen Masse erstarrt. Das Produkt wird in einer Reibschale zerkleinert und 4 Stunden bei 50°C im Vakuumtrockenschrank getrocknet. Die Ausbeute ist quantitativ (>99%). Das so erhaltene Kreatin-pyruvat (1:2) schmilzt bei 90 bis 95°C unter Zersetzung (Kapillare).

### Beispiel 4

29,8 g (0,2 mol) Kreatin-Monohydrat werden mit 8,8 g (0,1 mol) Brenztraubensäure unter Zusatz von 20 ml Tetrahydrofuran in einer Reibschale vermischt. Das Gemisch wird zunehmend zäher und erstarrt schließlich zu einer weißen, feinkristallinen Masse. Das Produkt wird 4 Stunden bei 50°C im Vakuumtrockenschrank getrocknet. Die Ausbeute ist quantitativ (99%). Das Kreatin-pyruvat (2:1) schmilzt bei 118 bis 120°C unter Zersetzung (Kapillare).

## Patentansprüche

1. Kreatin-pyruvate der allgemeinen Formel (I)
(Kreatin)ₓ(Pyruvat)_{y}(H₂O)ₙ (I)
wobei
x = 1 bis 100
y = 1 bis 10 und
n = 0 bis 10 bedeuten.

2. Kreatin-pyruvate nach Anspruch 1,
**dadurch gekennzeichnet,**
daß x = 1 bis 5, y = 1 bis 2 und n = 0 bis 2 bedeuten.

3. Kreatin-pyruvate nach einem der Ansprüche 1 und 2,
**dadurch gekennzeichnet,**
daß das Pyruvat-Anion als 2,2-Dihydroxypropionat-Anion vorliegt.

4. Verfahren zur Herstellung der Kreatin-pyruvate nach einem der Ansprüche 1 bis 3,
**dadurch gekennzeichnet,**
daß man Brenztraubensäure und Kreatin im Molverhältnis Kreatin zu Brenztraubensäure von 100 : 1 bis 1 : 10 bei Temperaturen von -10 bis 90 °C umsetzt.

5. Verfahren nach Anspruch 4,
**dadurch gekennzeichnet,**
daß das Molverhältnis von Kreatin zu Brenztraubensäure 5 : 1 bis 1 : 2 beträgt.

6. Verfahren nach einem der Ansprüche 4 und 5,
**dadurch gekennzeichnet,**
daß die Umsetzung bei Temperaturen zwischen 10 und 30°C erfolgt.

7. Verfahren nach einem der Ansprüche 4 bis 6,
**dadurch gekennzeichnet,**
daß die Umsetzung in Gegenwart eines polaren Lösungsmittels durchgeführt wird.

8. Verfahren nach Anspruch 7,
**dadurch gekennzeichnet,**
daß man als Lösemittel Alkohole, Ether, Ketone, Ester oder Gemische davon verwendet.

9. Physiologisch verträglicheZusammensetzungen, die Kreatin-pyruvate nach einem der Ansprüche 1 bis 3 zusammen mit mindestens einer weiteren physiologisch verträglichen Substanz ausgewählt aus der Gruppe umfassend pharmazeutische Hilfs- oder Trägerstoffe, Vitamine, Mineralstoffe, Kohlenhydrate, Aminosäuren oder andere Nahrungsergänzungsmittel enthalten.

10. Verwendung von Kreatin-pyruvaten nach einem der Ansprüche 1 bis 3 zur Herstellung eines Mittels für die Steigerung der Ausdauer und Kraft im Sportbereich, für die Gewichts- und Körperfettreduzierung im Gesundheitsbereich, bei der Behandlung von Sauerstoffmangelzuständen (lschämie), Fettsucht und Übergewicht, und für das Fangen von Radikalen sowie als Nahrungsmittelergänzungszusatz.

## Claims

1. Creatine pyruvates with the general formula (I)
(creatine)ₓ(pyruvate)_{y}(H₂O)ₙ (I)
where
x = 1 to 100
y = 1 to 10 and
n = 0 to 10

2. Creatine pyruvates as claimed in claim 1,
**wherein**
x = 1 to 5, y = 1 to 2 and n = 0 to 2.

3. Creatine pyruvates according to one of claims 1 and 2,
**wherein**
the pyruvate anion is present as 2,2-dihydroxypropionate anion.

4. A method of producing creatine pyruvates according to one of claims 1 to 3,
**wherein**
pyruvic acid and creatine are reacted in a molar ratio of 100 : 1 to 1 : 10 creatine to pyruvic acid at temperatures of -10 to 90°C.

5. A method according to claim 4,
**wherein**
the molar ratio of creatine to pyruvic acid is 5: 1 to 1 : 2.

6. A method according to one of claims 4 and 5,
**wherein**
the reaction is carried out at temperatures between 10 and 30°C.

7. A method according to one of claims 4 to 6,
**wherein**
the reaction is carried out in the presence of a polar solvent.

8. A method according to claim 7,
**wherein**
alcohols, ethers, ketones, esters or mixtures thereof are used as solvent.

9. Physiologically compatible compositions which contain the creatine pyruvates according to one of claims 1 to 3 and at least one additional physiologically compatible substance selected from the group comprising pharmaceutical adjuvants or carriers, vitamins, mineral substances, carbohydrates, amino acids or other food supplement additives.

10. The use of creatine pyruvates according to one of claims 1 to 3 for production of an agent for enhancing stamina and strength in the field of sport, for reducing weight and body fat in the field of health, in the treatment of conditions of oxygen deficit (ischemia), obesity and overweight, and as a radical interceptor and a food supplement additive.

## Revendications

1. Pyruvates de créatine de formule générale (I)
(créatine)ₓ(pyruvate)_{y}(H₂O)ₙ (I)
où
x = 1 à 100
y = 1 à 10 et
n = 0 à 10.

2. Pyruvates de créatine selon la revendication 1 caractérisés en ce que x = 1 à 5, y = 1 à 2 et n = 0 à 2.

3. Pyruvates de créatine selon l'une des revendications 1 et 2 caractérisés en ce que l'anion pyruvate est sous forme d'anion 2,2-dihydroxypropionate.

4. Procédé de préparation des pyruvates de créatine selon l'une des revendications 1 à 3 caractérisé en ce que l'on fait réagir de l'acide pyruvique et de la créatine dans le rapport molaire de la créatine à l'acide pyruvique de 100:1 à 1:10 à des températures de -10 à 90°C.

5. Procédé selon la revendication 4 caractérisé en ce que le rapport molaire de la créatine à l'acide pyruvique est de 5:1 à 1:2.

6. Procédé selon l'une des revendications 4 et 5 caractérisé en ce que la réaction a lieu à des températures situées entre 10 et 30°C.

7. Procédé selon l'une des revendications 4 à 6 caractérisé en ce que la réaction est conduite en présence d'un solvant polaire.

8. Procédé selon la revendication 7 caractérisé en ce que l'on utilise comme solvant des alcools, des éthers, des cétones, des esters ou des mélanges de ceux-ci.

9. Compositions physiologiquement acceptables qui contiennent des pyruvates de créatine selon l'une des revendications 1 à 3 en même temps qu'au moins une autre substance physiologiquement acceptable choisie dans le groupe comprenant les adjuvants et supports pharmaceutiques, les vitamines, les substances minérales, les glucides, les acides aminés et d'autres compléments pour l'alimentation.

10. Utilisation de pyruvates de créatine selon l'une des revendications 1 à 3 pour la préparation d'un agent pour augmenter l'endurance et la force dans le domaine des sports, pour la réduction du poids et des graisses corporelles dans le domaine de la santé, dans le traitement des états de déficit en oxygène (ischémie), de l'obésité et de l'excès de poids, et pour le piégeage de radicaux ainsi que comme additif de complémentation pour aliments.
